# EUROPEAN PATENT APPLICATION

(11) **EP 3 852 115 A1**
(43) Date of publication of application: **21.07.2021**
(21) Application number: 21151506.9
(22) Date of filing: 14.01.2021
(51) Int. Cl.: G16H 10/20, G16H 10/60

(54) **COMPUTER-IMPLEMENTED METHOD FOR COLLECTING EXPERIMENTAL AND/OR CLINICAL DATA**

(30) Priority: 17.01.2020 CH 532020; 17.01.2020 IT 202000000862
(71) Applicant: Medical Trials Analysis Swiss SA, 6900 Lugano (CH)
(72) Inventor: Morelli, Cristina, 6965 Lugano (CH); Tintori, Andrea, 21040 Origgio (IT)
(74) Representative: PGA S.p.A., Milano, Succursale di Lugano

(57) **Abstract**

The present disclosure comprises a computer-implemented method for collecting trial and/or clinical data from at least a patient subject (100), the method comprising:
- a phase of proposing and/or generating a user interface (10) on a first electronic device (300) operatively associated to a first patient subject (100), wherein the user interface (10) comprises at least a data field (11) configured and dedicated to the introduction, by the first patient subject (100), of at least a first type of trial and/or clinical electronic data relating to the first patient subject (100);
- a phase of reception (1003) on a proposing electronic computer (20; 40) at least of said first type of electronic data relating to the first patient subject (100) from the first electronic device (300), and a subsequent intermediate and/or temporary phase of storage of at least part of said first type of electronic data of the first patient subject (100), thus received, on an electronic memory (12, 42),
- a phase of electronic validation (1007), performed by an evaluating subject (200) distinct with respect to the patient subject (100) and subsequent in time to said phase of reception (1003), wherein the evaluating subject (200) performs a verification phase on at least part of the said first type of electronic data of the first patient subject (100) and a phase of generation and/or transmission, through a first electronic computer (20) operatively associated and destined to the evaluating subject (200), of an electronic command of confirmation of correctness and/or applicability of at least a part of said first type of electronic data of the first patient subject (100),
said electronic command of confirmation causing a final storage (1008) of a correct part of said first type of electronic data of the first patient subject (100) for which the correctness and/or applicability has been electronically confirmed by the electronic command of confirmation.

## Description

### FIELD OF THE INVENTION

The present invention relates to the field of computer programs, concerns the field of health data processing methods and the field of electronic devices and systems configured to process data.

### BACKGROUND

The clinical trial is a phase intended to collect data from one or more patients in order to establish determined characteristics, safety profiles and drug effects. This clinical trial phase is a legally required phase, in order to allow the introduction of drugs into the market. Traditionally the clinical trial is conducted by a (medical) experimenter through a plurality of medical examinations of the patients, from whom the medical experimenter collects information about the effects of the drug, and in particular, but not only, about the therapeutic efficacy and adverse events, and on the basis of these information fills out the data collection report forms that are eventually sent to the pharmaceutical company that intends to produce the drug.

The pre-marketing clinical trial phase can be followed by further clinical research phases carried out at a later stage, wherein more information is collected; this additional information can be useful to complete the clinical picture of the drug and/or identify any interactions or adverse effects that may occur during the ordinary treatment or during the post-treatment phase.

It is also known that there is a category of drugs whose action and/or half-life are particularly rapid, and for which the occurrence of adverse effects or anyway the therapeutic effect occurs within an extremely short time.

The Applicant has realized that in particular for these categories of drugs whose action and/or half-life are rapid, but in general for the whole category of drugs which are then actually placed on the market, the patient's report to the experimenter is affected by some disadvantages: among these disadvantages there is, first of all, a slight possibility to identify precisely the moment in which certain effects, whether adverse or not, occur on the patient itself. To date, the precision with which those data can be collected is exclusively based on the memory of the patient and it is therefore difficult for the patient to precisely recollect when, or worse, if, certain effects have occurred. This disadvantage is especially present when the interview with the experimenter takes place after a significant period of time with respect to the moment when these effects occurred.

The Applicant also notes that the clinical trial of certain drugs can be carried out on elderly patients. For this category of patients, a precise collection of information about the effects of the drug must take place as soon as possible, in particular as this category of patients is more likely to have significantly poorer memory than young subjects.

The purpose of the present disclosure is to describe a method, a distributed computing environment and a computer program for solving the aforementioned drawbacks, and in particular for obtaining a precise and rapid collection of data in order to conduct a clinical trial of one or more drugs.

### SUMMARY

The object of the present disclosure will now be described in accordance with certain salient aspects, which can be combined among each other and/or can be combined with portions of the following detailed description and/or with the attached claims. Where combinability or dependencies among aspects are defined, these are to be understood as not limiting.

According to a first aspect, it is described a computer-implemented method for collecting trial and/or clinical data from at least one patient subject (100), the method comprising:
- a phase of proposing and/or generating a user interface (10) on a first electronic device (300) operatively associated to a first patient subject (100), wherein the user interface (10) comprises at least a data field (11) configured and destined to the introduction, by the first patient subject (100), of at least a first type of trial and/or clinical electronic data relating to the first patient subject (100);
- a phase of reception (1003) on a proposing electronic computer (20; 40) of at least said first type of electronic data relating to the first patient subject (100) from the first electronic device (300), and a subsequent phase of intermediate and/or temporary storage of at least part of said first type of electronic data of the first patient subject (100), so received, on an electronic memory (12, 42),
- a phase of electronic validation (1007), performed by an evaluating subject (200) distinct from the patient subject (100) and subsequent in time to said phase of reception (1003), wherein the evaluating subject (200) carries out a phase of verification on at least part of said first type of electronic data of the first patient subject (100) and a phase of generation and/or transmission, through a first electronic computer (20) operatively associated and destined to the evaluating subject (200), of an electronic command of confirmation of correctness and/or applicability of at least a part of said first type of electronic data of the first patient subject (100),
said electronic command of confirmation causing a final storage (1008) of a correct part of said first type of electronic data of the first patient subject (100) for which the correctness has been electronically confirmed through the electronic command of confirmation.

For the purposes of this disclosure, "final storage" means a storage performed after the electronic command of confirmation, and such storage is not necessarily intended as storage not limited in time.

According to a further non-limiting aspect, the electronic command of confirmation causes an identification of a correct part of said first type of electronic data of the first patient subject (100).

According to a further non-limiting aspect, the electronic command causes a final storage (1008) of the correct part of the said first type of electronic data of the first patient subject (100) on the first electronic computer (20).

According to a further non-limiting aspect, the electronic command causes a final storage (1008) of the correct part of said first type of electronic data of the first patient subject (100) on a second electronic computer (40), different from the first electronic computer (20), optionally operatively associated to a pharmaceutical company (400). According to a further non-limiting aspect, the intermediate and/or temporary storage is a first storage, while the final storage is a second storage and/or a confirmation of the first storage.

According to a further non-limiting aspect, the selective transmission of electronic data is a selectively activated electronic data transmission towards the first electronic device (300). According to a further non-limiting aspect, the selective transmission of electronic data towards the first electronic device (300) takes place between the proposing electronic computer (20; 40) and said first electronic device (300).

According to a further non-limiting aspect, the electronic proposition of the user interface (10) towards the specific electronic device (300) takes place between the proposing electronic computer (20; 40) and the specific electronic device (300).

According to a further non-limiting aspect, the selective transmission of electronic data towards the first electronic device (300) is preceded by a phase of setting-up a logical channel between the proposing electronic computer and the first electronic device (300); optionally the method comprising the keeping-up of the logical channel for a predetermined period of time of which the end of a data retransmission from the electronic device (300) towards the proposing electronic computer (20; 40) determines a limit.

According to a further non-limiting aspect, the proposing electronic computer (20; 40) is the first electronic computer (20).

Alternatively, according to a further non-limiting aspect, the proposing electronic computer (20; 40) is a second electronic computer (40), different from the first electronic computer (20), said proposing electronic computer (20; 40) being distinct from the first electronic computer (20).

According to a further non-limiting aspect, the phase of reception (1003) on the proposing electronic computer (20; 40) of at least said first type of electronic data relating to the first patient subject (100) comprises their at least temporary storage on the first electronic computer (20) operatively associated with said evaluating subject (200).

According to a further non-limiting aspect, the electronic memory (12) is the electronic memory of said first electronic computer (20).

According to a further non-limiting aspect, the electronic memory (12) is an electronic memory operatively accessible by the first electronic computer (20).

According to a further non-limiting aspect, the second electronic computer (40) is managed or owned by the pharmaceutical company (440) and/or is physically located in the pharmaceutical company (440) and/or is an electronic computer managed by a third party on behalf of said pharmaceutical company (440).

According to a further non-limiting aspect, the electronic memory (42) is the electronic memory of the second electronic computer (40).

According to a further non-limiting aspect, the phase of electronic validation (1007) comprises, optionally ends with, an automatic transmission of the correct part of said first type of trial and/or clinical electronic data relating to the first patient subject (100) on the first electronic computer (20) operatively associated to said evaluating subject (200), and/or on a second electronic computer (40), different from the first electronic computer (20), optionally operatively associated to a pharmaceutical company; the automatic transmission taking place automatically following the electronic command of confirmation.

According to a further non-limiting aspect, following the phase of electronic validation (1007), a non-correct part of said first type of electronic data of the first patient subject (100) is defined, wherein said non-correct part is a part of the first type of electronic data of the first patient subject (100) for which the electronic command of confirmation of correctness and/or applicability has not been transmitted, and said non-correct part is electronically identified and/or discarded and, optionally, is electronically identified and/or discarded without electronic erasing and/or is ended with an electronic archiving separate from the non-correct part of the first type of electronic data relating to the first patient subject (100).

According to a further non-limiting aspect, the method comprises a phase of electronic selection of at least the first electronic device (300), among a plurality of electronic devices (300) each one operatively associated to a respective patient subject (100), optionally through the first electronic computer (20), and the proposition and/or generation of the user interface (10) on the first electronic device (300) comprises a selective transmission of electronic data towards the first electronic device (300).

According to a further non-limiting aspect, the method comprises a phase of electronic distinction (1004) between the first type of trial and/or electronic data and a second type of subjective electronic data, said phase of distinction taking place as a result of the phase of reception (1003) of the first and second type of subjective electronic data and optionally taking place before the phase of electronic validation (1007).

According to a further non-limiting aspect, the phase of electronic distinction (1004) is performed by the first electronic computer (20) and/or by the second electronic computer (40) on the basis of a predefined data processing scheme.

According to a further non-limiting aspect, the electronic proposition (1001) of the user interface (10) takes place through a transmission of electronic data of a client-server type, wherein the proposing electronic computer (20; 40), in particular the first electronic computer (20) and/or the second electronic computer (40), acts as a server, and/or wherein the first electronic device (300) acts as a client, the first electronic device (300) being provided with a monitor, said electronic data being configured to cause the appearance on the monitor of at least a screen from which the patient subject (100) identifies questions to answer through the introduction of electronic data inside at least one data field (11).

According to a further non-limiting aspect, the electronic proposition (1001) of the user interface towards said patient subject (100), comprises the transmission of display electronic data which cannot be altered by the patient subject (100).

According to a further non-limiting aspect, the phase of electronic proposition (1001) of the user interface comprises a transmission of display electronic data towards a remote electronic device, operatively accessible by the patient subject (100).

According to a further non-limiting aspect, the method comprises the definition of a distributed computing environment and/or of a closed-type logic network of data transceiving, wherein the interaction of one or more patient subjects (100) with one or more evaluating subjects (200) takes place only following a phase of introduction of access credentials and verification of the correctness thereof, at the moment of the introduction by the patient subject (100) and/or of the evaluating subject (200).

According to a further non-limiting aspect, the first type of trial and/or clinical electronic data comprises at least one of the data of the following list:
- a datum of motivation for consuming a drug;
- a datum comprising the indication for which the patient subject (100) decides to start a treatment cycle;
- a datum of localization of pain felt by the patient subject (100);
- a datum comprising a pain level perceived by the patient subject (100) before starting the treatment with a drug,
- a datum comprising the number of capsules consumed by the patient subject (100);
- a datum comprising a level of pain perceived by the patient subject (100) following the beginning of the treatment by means of a drug, and the level of pain perceived at substantially predefined time instants starting from the beginning of the treatment;
- a datum of general satisfaction of the patient subject (100) at the end of a treatment with a drug;
- a datum of consumption of further and/or other drugs with respect to said drug, optionally drugs with anti-inflammatory action;
- a datum comprising a level of pain perceived by the patient subject (100) following the end of the treatment by means of a drug;
- a datum comprising a number of elements of drug consumed by the patient subject (100) and/or an amount of drug consumed by the patient;
- a datum comprising the potential presence of adverse events;
- a datum comprising the potential presence of serious adverse events;
- a datum including the number of days in which the drug is consumed;
- a datum comprising consumptions of a drug for off-label applications;
- a datum comprising the potential consumption of the drug at intervals lower than 4 hours;
- a datum comprising the potential consumption of the drug at too short cycles.

According to a further non-limiting aspect, the level of perceived pain is expressed and/or transmitted and/or entered by the patient subject (100) as a numeric electronic datum, measured on a predefined value VAS scale.

According to a further non-limiting aspect, the method comprises a phase of time definition of a first time instant (t0) of the beginning of the treatment, and of storage of said time instant in a memory (12) of an electronic computer which is operatively accessible by the evaluating subject (200) and/or by the first electronic computer (20) operatively connected to the evaluating subject (200).

According to a further non-limiting aspect, the method comprises a phase of electronic filtering (1005) of said first type of electronic trial and/or clinical data relating to the first patient subject (100) and received with said phase of reception (1003), wherein the electronic filtering (1005) takes place in accordance to a predetermined risk criterion.

According to a further non-limiting aspect, in the phase of electronic filtering (1005), in case in the phase of reception following the proposition of the user interface (10) towards the first electronic device (300) and/or the generation of the user interface (10) on the first electronic device (300), the first patient subject (100) through the first electronic device (300) should transmit an electronic datum relating to serious adverse events, the method comprises a phase of automatically transmission of:
- a priority alerting message (1006) towards a pharmaceutical company (400) and/or towards a third company, differing from said pharmaceutical company (400), in particular towards the second electronic computer (40) operatively associated to the pharmaceutical company (400), and/or
- a priority alerting message towards the evaluating subject (200), in particular towards the first electronic computer (20) operatively associated to said evaluating subject (200), and/or
- a priority alerting message towards a medical reviewer or a project manager (500), in particular towards a third electronic computer operatively associated to the medical reviewer or project manager (500);
and/or wherein the method comprises an immediate electronic proposition of an emergency user interface towards the first electronic device (300), so that the user subject (100) can transmit further detailed electronic data relating to serious adverse events towards the evaluating subject (200) and/or the pharmaceutical company (400) and/or the medical reviewer or project manager (500).

For the purposes of this disclosure, "serious adverse event" (SAE) mean the occurrence, during the participation in a clinical trial, of any adverse effect resulting in one or more of the consequences of this list: a death of the patient subject (100), an optionally actual and/or immediate risk to the life of the patient subject (100), a hospitalization of the patient subject (100) or a prolongation of existing hospitalization of the patient subject (100), a persistent and/or significant disability or incapacity of the patient subject (100), a congenital anomaly and/or a birth defect, an operation to prevent permanent damage or impairment of the patient subject (100).

For the purposes of this disclosure, it is not to be considered as "serious adverse event" an effect or event that hypothetically could have caused death if it had been more severe.

According to a further non-limiting aspect, the electronic proposition (1001) of the user interface takes place following the phase of time definition of the first time instant (t0) of the beginning of the treatment.

According to a further non-limiting aspect, the method comprises a phase of transmission of an electronic alerting message (1006) towards the first electronic device (300) operatively associated to the patient subject (100), optionally the transmission taking place through the user interface, and a subsequent phase of electronic waiting for a response provided by said patient subject (100).

According to a further non-limiting aspect, the transmission of the electronic alerting message takes place in correspondence of at least a predetermined and second time instant (t1) distinct from the first time instant (t0).

According to a further non-limiting aspect, the method comprises an electronic calculation of a time shift from the first time instant (t0) to the second time instant (t1) and comprises, when the second time instant (t1) is reached, the automatic and optionally immediate carrying out of the transmission of the alerting message.

According to a further non-limiting aspect, said electronic message contains a request for the transmission of an electronic data comprising a level of pain perceived by said patient subject (100).

According to a further non-limiting aspect, the method comprises, at the end of said treatment, a phase of electronic delivery of a user interface towards the first electronic device (300) wherein, through said at least one user interface, a message comprising at least one of the data included in the following list is presented:
- a datum requesting whether the patient subject (100) has consumed further drugs for the treatment of pain,
- a datum requesting the degree of subjective satisfaction with the consumption of the drug;
- a datum of whether the patient subject (100) experienced an adverse event;
- a confirmation datum of drug consumption, said datum of confirmation being presented on said user interface only when the patient subject (100) has never transmitted any electronic data.

According to a further non-limiting aspect, the electronic control causes a final storage (1008) of a correct part of the electronic data forming part of the first type of electronic data on a memory (42) operatively associated to and/or accessible by the second electronic computer (40).

According to a further non-limiting aspect, the method comprises a phase of definition of a subnet of accessibility to the memory (42) that is operatively accessible by and/or associated to at least the second electronic computer (40), wherein said subnet, in writing mode, comprises the first electronic computer (20), the memory (42) itself and the second electronic computer (40) and excludes at least the first electronic device (300) associated to the patient subject (100), optionally said subnet including, in writing mode, exclusively the first electronic computer (20), the memory (42) itself and the second electronic computer (40).

According to a further non-limiting aspect, the method comprises a phase of definition of a subnet of accessibility to the memory (42) operatively accessible by and/or associated to at least the second electronic computer (40), wherein said subnet, in reading mode, comprises the second electronic computer (40), the memory (42) itself and excludes at least the first electronic device (300) associated to the patient subject (100), optionally said subnet including, in reading mode, exclusively the memory (42) and the second electronic computer (40).

According to a further non-limiting aspect, the accessibility subnet is a logical subnet.

According to a further non-limiting aspect, the user interface is an electronic interface of the guided type and/or is an electronic interface with hierarchical structure, configured to allow the patient subject (100) to navigate on selection structures nested and/or alternatively and/or selectively selectable among themselves, optionally through an action on virtual buttons represented on a monitor of the first electronic device (300).

According to a further non-limiting aspect, the phase of reception (1003) comprises a phase of selective distribution, optionally automatic and/or immediate, of at least part of the electronic data received from the first electronic device (300) operatively associated to the patient subject (100), wherein in the selective distribution there is a selective, optionally automatic and/or immediate retransmission of at least part of the electronic data received, towards a plurality of distinct recipients, optionally wherein each at least a first and a second recipient of the aforementioned plurality receive a same part of said electronic data.

According to a further aspect, a distributed computing environment is described herein, said computing environment comprising:
- at least a proposing electronic computer (20; 40), configured for causing a proposition and/or generation of, and/or an operative association with, a user interface (10) towards and/or on at least a first electronic device (300) operatively associated to a first patient subject (100) and remotely positioned with respect to the proposing electronic computer (20; 40), wherein the user interface (10) comprises at least a data field (11) configured and destined to the introduction, by the first patient subject (100), of at least a first type of trial and/or clinical electronic data;
- at least a first electronic computer (20) operatively associated to an evaluating subject (200) different with respect to the patient subject (100), and an electronic memory (12, 42) operatively accessible by the first electronic computer (20), wherein the first electronic computer (20) is configured for receiving (1002) at least said first type of trial and/or clinical electronic data transmitted by the patient subject (100) through the first electronic device (300), and is further configured to cause an intermediate and/or temporary storage of the electronic data received on the electronic memory (12, 42),
wherein:
- the first electronic computer (20) is configured for causing an electronic validation (1007), operatively performed by the evaluating subject (200), the electronic validation (1007) being subsequent in time to the reception (1003) of the electronic data, wherein the evaluating subject (200) performs an action of verification on at least part of the first type of trial and/or clinical electronic data of the first patient subject (100) and wherein the first electronic computer (20) is configured for transmitting an electronic command of confirmation of correctness and/or applicability of at least a part of said first type of electronic data of the first patient subject (100), said electronic command of confirmation causing a final storage (1008) of a correct part of said first type of electronic data of the first patient subject (100) for which the correctness and/or applicability has been electronically confirmed through the electronic command of confirmation.

According to a further non-limiting aspect, the proposing electronic computer corresponds to and/or comprises the first electronic computer (20).

According to a further non-limiting aspect, the distributed computing environment comprises a database wherein it is stored at least an electronic profile operatively associated to a first patient subject (100) and/or to a first electronic device (300) in its turn operatively associated to a first patient subject (100).

According to a further non-limiting aspect, the selective transmission is a transmission aimed at allowing at least said electronic proposition of the user interface towards the first electronic device (300).

According to a further non-limiting aspect, the distributed computing environment is configured to cause the keeping-up of said logic channel for a predetermined period of time determined by a retransmission of data from the electronic device (300) towards the proposing electronic computer.

According to a further non-limiting aspect, the distributed computing environment is configured to allow a selection of at least a specific user subject (100) and to cause a selective transmission of data and/or a selective proposition of the user interface towards said at least a first user subject (100).

According to a further non-limiting aspect, the distributed computing environment is configured to establish a logic channel between the proposing electronic computer (20; 40), optionally the first electronic computer (20), and the first electronic device (300), wherein said logic channel is a selective transmission logic channel between the proposing electronic computer and said first electronic device (300).

According to a further non-limiting aspect, the distributed computing environment is a closed-type computing environment, wherein the interaction of one or more patient subjects (100) with one or more evaluating subjects (200) takes place only following a phase of introduction of credentials of access and verification of the correctness thereof, this verification being carried out automatically by the distributed computing environment itself following said phase of introduction.

According to a further non-limiting aspect, the first electronic computer (20) is configured to transmit the correct part of said first type of electronic data of the first patient subject (100) to a second electronic computer (40), different than the first electronic computer (20), optionally wherein the second electronic computer (40) is operatively associated to a pharmaceutical company (400) and/or to a third company, different than said pharmaceutical company (400).

According to a further non-limiting aspect, the first electronic computer (20) is configured to cause a final storage (1008) of the correct part of said first type of electronic data of the first patient subject (100) on a second electronic computer (40), optionally operatively associated to a pharmaceutical company (400).

According to a further non-limiting aspect, the first computer is configured to cause a final storage (1008) of the correct part of the electronic data through said electronic command of confirmation.

According to a further non-limiting aspect, the distributed computing environment comprises a second electronic computer (40), different than the first electronic computer (20), said second electronic computer (40) being operatively associated to a pharmaceutical company (400) and/or to a third company different from said pharmaceutical company (400); the second electronic computer (40) being configured at least to receive the correct part of the first type of electronic data of the first patient subject (100) transmitted by the first electronic computer (20) when the final storage (1008) takes place.

According to a further non-limiting aspect, the distributed computing environment comprises an electronic memory (42), operatively associated to said second electronic computer (40).

According to a further non-limiting aspect, the second electronic computer (40) is configured to store said correct part of the first type of trial and/or clinical electronic data of the first patient subject (100) from the first electronic computer (20) towards the memory (42) operatively accessible by and/or associated to at least the second electronic computer (40).

According to a further non-limiting aspect, the memory (42) operatively accessible by and/or associated to at least the second electronic computer (40), is not electronically accessible by any patient subject (100).

According to a further non-limiting aspect, the distributed computing environment defines an subnet of accessibility to the memory (42) operatively accessible by and/or associated to at least the second electronic computer (40), wherein said subnet, in writing mode, comprises the first electronic computer (20), the memory (42) itself and the second electronic computer (40) and excludes at least the first electronic device (300) associated to the first patient subject (100), optionally said subnet including, in writing mode, exclusively the first electronic computer (20), the memory (42) itself and the second electronic computer (40).

According to a further non-limiting aspect, the distributed computing environment defines a subnet of accessibility to the memory (42) operatively accessible by and/or associated to at least the second electronic computer (40), wherein said subnet, in reading mode, comprises the second electronic computer (40), the memory (42) itself and excludes at least the first electronic device (300) associated to the first patient subject (100), optionally said subnet including, in reading mode, exclusively the memory (42) and the second electronic computer (40).

According to a further non-limiting aspect, the first electronic computer (20) is configured to end the electronic validation (1007) with an automatic transmission of the correct part of said first type of trial and/or clinical electronic data on the second electronic computer (40), optionally operatively associated to the pharmaceutical company (400).

According to a further non-limiting aspect, the distributed computing environment is configured to define a non-correct part of the first type of clinical trial electronic data, and/or is configured to make an electronic distinction between a non-correct part of the first type of trial and/or clinical electronic data with respect to the correct part of the first type of clinical trial electronic data, wherein said non-correct part of the first type of trial and/or clinical electronic data is a part of said data for which an electronic command of confirmation of correctness and/or applicability has not been transmitted, and the first electronic computer (20) is configured, during the electronic validation (1007), to identify and/or discard this non-correct part of said first type of trial and/or clinical electronic data, and, optionally, is configured to end the intermediate storage with a separate electronic archiving of the non-correct part of the first type of trial and/or clinical electronic data relating to the first patient subject (100).

According to a further non-limiting aspect, the first electronic computer (20) is configured to perform an electronic distinction (1004) between the first type clinical trial electronic data, and a second type of subjective electronic data, and is configured to carry out the electronic distinction (1004) following the reception (1002) of said first type of trial and/or clinical electronic data and said second type of subjective electronic data transmitted by the first electronic device (300), and is configured to carry out the electronic distinction (1004), optionally, before the electronic validation (1007).

According to a further non-limiting aspect, the second electronic computer (20) is configured to cause the electronic proposition on a user interface of the evaluating subject (200) only of the first type of clinical trial electronic data, so that the evaluating subject (200) can complete the electronic validation only in relation to the data of said first type of clinical trial electronic data.

According to a further non-limiting aspect, the first electronic computer (20) is configured to receive from the first electronic device (300) operatively associated to the patient subject (100), a datum of the level of perceived pain; said datum of the level of perceived pain being a purely numerical electronic datum, measured on a predefined value VAS scale.

According to a further non-limiting aspect, the distributed computing environment is configured to store a first time instant of the beginning of the treatment of the patient subject (100) in a memory that is operatively accessible by the evaluating subject (200) and/or operatively accessible by the first electronic computer (20).

According to a further non-limiting aspect, the distributed computing environment is configured to perform an electronic filtering (1005) of the data transmitted by the patient subject (100) through the first electronic device (300) towards the first electronic computer (20), said electronic filtering taking place in accordance to a predetermined risk criterion.

According to a further non-limiting aspect, the patient subject (100) through the first electronic device (300) should transmit an electronic datum relating to serious adverse events, said electronic filtering (1005) causes the transmission of, and/or the first electronic computer (20) is configured to transmit:
- a priority alerting message (1006) towards a pharmaceutical company (400), in particular towards the second electronic computer operatively associated to the pharmaceutical company (400), and/or
- a priority alerting message towards the evaluating subject (200), in particular towards the first electronic computer operatively associated to said evaluating subject, and/or
- a priority alerting message towards a medical reviewer or a project manager (500), in particular towards a third electronic computer operatively associated to the medical reviewer or project manager (500); and/or
- an immediate electronic proposition of an emergency user interface towards the electronic device (300), so that the user subject (100) can transmit further detailed electronic data relating to serious adverse events towards the evaluating subject (200) and/or the pharmaceutical company (400) and/or the medical reviewer or project manager (500).

According to a further non-limiting aspect, the proposing electronic computer is configured for to electronically proposing (1001) the user interface following the definition of the first time instant (t0) of the beginning of the treatment.

According to a further non-limiting aspect, the distributed computing environment is configured to transmit an alerting electronic message (1006) towards the first electronic device (300) operatively associated to the patient subject (100), optionally the transmission taking place through the proposing electronic computer, and to wait for a response provided by said patient subject (100) through the first electronic device (300).

According to a further non-limiting aspect, the distributed computing environment is configured to transmit the alerting electronic message in correspondence of at least a predetermined and second time instant (t1) different with respect to the first time instant (t0).

According to a further non-limiting aspect, the distributed computing environment is configured to perform an electronic calculation of a time sliding from the first time instant (t0) to the second time instant (t1) and comprises, at the moment of reaching the second time instant (t1), the automatic and optionally immediate execution of the transmission of the alerting message.

According to a further non-limiting aspect, the distributed computing environment is configured to transmit through the proposing electronic computer, at the end of said treatment, a user interface towards the electronic device (300) wherein through said at least a user interface a message is presented comprising at least one of the data comprised in the following list:
- a datum requesting whether the patient subject (100) has consumed further drugs for the treatment of pain,
- a datum requesting the degree of subjective satisfaction with the consumption of the drug;
- a datum of whether the patient subject (100) has experienced an adverse event;
- a drug consumption confirmation datum, said confirmation datum being presented on said user interface only when the patient subject (100) has never transmitted any electronic datum.

According to a further non-limiting aspect, the proposing electronic computer is configured to transmit a guided-type user interface and/or a user interface with hierarchical structure at least towards the first electronic device (300), the user interface being configured for allowing the first patient subject (100) to navigate on selection structures nested and/or alternatively selectable and/or selectively selectable among themselves, optionally through an action on virtual buttons represented on a monitor of the electronic device (300).

According to a further non-limiting aspect, the computing environment is configured to receive (1003) electronic data, in particular said first type of clinical trial electronic data, from the first electronic device (300) operatively associated to the patient subject (100), and in accordance with a predetermined distribution scheme, cause a selective, optionally automatic and/or immediate retransmission of at least part of the received electronic data, in particular at least part of said first type of clinical trial electronic data, to a plurality of distinct recipients.

According to a further non-limiting aspect, the computing environment is configured to transmit electronic data, optionally comprising data of said first type and/or said second type, at least towards a first and a second recipient, optionally at least towards a first and a second electronic device (300), each operatively associated to a respective first patient subject (100) and second patient subject (100), the computing environment being configured to cause a transmission of electronic data so that each said first and said second recipient can receive a same part of said electronic data.

According to a further aspect, a software program is described, comprising portions of software code which when executed by a computer cause the execution of a method according to one or more of the present aspects.

According to a further aspect, a memory support is described, storing the software program according to the preceding aspect.

According to a further aspect, the use of the method and/or the use of the distributed computing environment for the collection of trial and/or clinical data relating to one or more drugs is described.

According to a further non-limiting aspect, a software application is described, configured to be installed on an electronic device (300), optionally a portable electronic device (300) supplied to a patient subject (100) participating in a clinical trial, the software application comprising portions of code which when executed by a data processing unit of the electronic device (300) cause:
- the appearance on a monitor of the electronic device (300) of a user interface (10) comprising at least a data field (11) configured and destined to the introduction, by the patient subject (100), of electronic data through the electronic device (300) and/or configured and destined to the selection of electronic data presented through the user interface itself;
- a transmission of at least part of the electronic data entered and/or selected by the patient subject (100) through the electronic device (300) towards an electronic computer remotely positioned with respect to the electronic device (300);
- a generation of at least an alerting or alarm signal, emitted by the electronic device (300), as a consequence of receiving an alarm notification emitted by said electronic computer.

According to a further non-limiting aspect, the electronic computer is a first electronic computer (20) operatively associated to an evaluating subject (200), said first electronic computer (20) optionally being the first electronic computer itself (20) which caused the data transmission.

According to a further non-limiting aspect, the software application comprises portions of software code which, when executed, cause the transmission of a signal of request of storage of a first time instant (t0) in a memory (12; 42) operatively accessible by the electronic computer, said first time instant corresponding to the time instant in which the patient subject (100) selects through the electronic device (300) a control or button (100b) of confirmation of the first consumption of a drug.

According to a further non-limiting aspect, the software application comprises portions of software code which, when executed, cause the generation of said alerting or alarm signal in correspondence to at least a predetermined time instant (t1) subsequent and decurrent from the first time instant (t0).

According to a further non-limiting aspect, following the transmission of the alerting or alarm signal, the electronic device (300) requires an action by the patient subject (100), optionally the confirmation of a datum of value of a datum of perceived pain level; said datum of perceived pain level being a purely numeric electronic datum, measured on a predefined value VAS scale.

### BRIEF DESCRIPTION OF THE DRAWINGS

The object of the present disclosure will be described hereinafter in preferred and non-limiting embodiments with reference to the attached figures, wherein:
- figure 1 shows a detailed diagram of the distributed computing environment that is the subject of the present disclosure;
- figure 2 shows a principle representation of a user interface electronically proposed to a patient subject who is subject to the collection of clinical trial data,
- figure 3 shows a flow chart that schematically represents the main phases of the method which is the subject of the present disclosure;
- figure 4 shows a detailed diagram representing the flow of information between the various parties involved in the collection of trial and/or clinical data according to the present disclosure;
- figure 5 shows a first screen of the proposed user interface with the distributed computing environment that is the subject of the present disclosure;
- figure 6 shows a second screen of the proposed user interface with the distributed computing environment that is the subject of the present disclosure;
- figure 7 shows a table where particular indications provided by a user subject are associated with moments related to the consumption of a drug.

### DETAILED DESCRIPTION

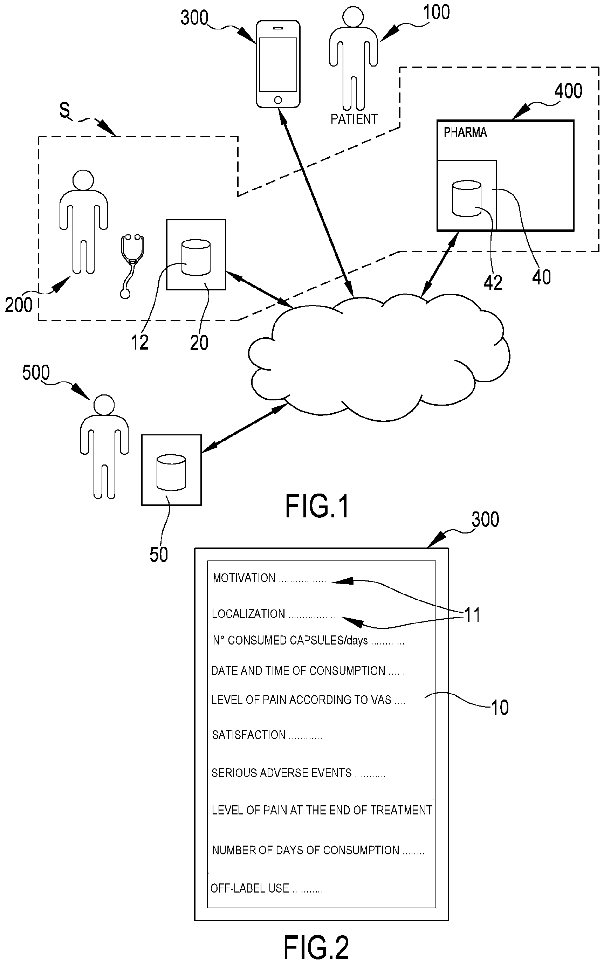

Figure 1 shows a simplified diagram of the elements forming part of the distributed computing environment forming the object of the present disclosure.

The subjects involved in the process of collection of trial and/or clinical data which forms the subject of the present disclosure comprise, in the first place, a first patient subject 100, represented in figure 1 individually but without this constituting a limitation. There is also at least an evaluating subject, identified with the reference number 200, whose purpose is to process and/or validate the trial and/or clinical data coming from one or more patient subjects 100, and in particular at least from the first patient subject 100. A further subject involved in the distributed computing environment forming the object of the present disclosure is represented by a pharmaceutical company or by a third party delegated by it, represented in figure 1 with the reference number 400.

Each of these subjects is equipped with an electronic laboratory of their own, equivalently, an electronic device of their own, intended to exchange data with other electronic laboratories or electronic devices, even remotely positioned. For this reason, in figure 1 the remotization of the various electronic devices or electronic computers is represented by the cloud positioned in the center of the figure itself. In particular, the first patient subject 100 is equipped with a (first) electronic device 300 of its own equipped with user interface means and, preferably and non-limiting thereto, it can be a smartphone device or a tablet PC, or an actual computer. In particular, for the purposes of the present disclosure, the patient subject 100 - electronic device 300 ratio is 1:1 in other words the first electronic device 300 is uniquely associated to the first patient subject 100. As it will become clearer from the following portion of description, the univocal association between the patient subject 100 and its electronic device 300 is conveniently given by a software application installed on the electronic device 300; therefore at least when the electronic device 300 is a smartphone device or a tablet PC, said electronic device 300 must not be understood as uniquely intended for the specific application described in the present disclosure. However, in an alternative and non-limiting embodiment, the electronic device 300 is a dedicated electronic device (so-called "application specific"). In this case, the aforementioned software application may take the shape of a firmware pre-installed on board of the electronic device 300 itself. According to a specific and non-limiting embodiment, the user interface means comprise at least a monitor, and elements to allow the introduction of electronic data by the patient subject 100. These elements can be included within the monitor, if of touch-sensitive type, or, alternatively or in combination, comprise a keyboard or an equivalent device.

The evaluating subject 200 is equipped with a first electronic computer 20 in its turn equipped with a memory 12; the pharmaceutical company 400 is in its turn equipped with a second electronic computer 40, in its turn equipped with a memory 42. It is to be noted that both the memory 12 and the memory 42 do not necessarily have to be inside the respective electronic computer, but can also be located externally to them, as long as they remain operatively accessible.

In the distributed computing environment that forms the object of the present disclosure, there is a proposing electronic computer, which is configured to electronically propose a user interface 10 towards the patient subject 100, in particular towards the electronic device 300 operatively associated to the patient subject 100. Preferably, but non-limiting thereto, the aforementioned proposing electronic computer comprises, or is, the first electronic computer 20 operatively associated to the evaluating subject 200. Alternatively, the proposing electronic computer comprises, or is, a second electronic computer distinct from the first.

The distributed computing environment object of the present disclosure is able to operate with at least a first type of data and preferably with two types of electronic data:
- a first type of electronic data, which comprise data of purely clinical nature required in the context of a clinical trial (also referred to as "first type of trial and/or clinical electronic data"),
- a second type of electronic data, which comprise subjective data expressed by the patient subject 100 towards the evaluating subject 200 within the same clinical trial.

The user interface 10 proposed through the proposing electronic computer, optionally the first electronic computer 20, is in particular a user interface proposed on a monitor of the electronic device 300 actively associated to the patient subject 100 through the opening of at least a logical channel between the proposing electronic computer and the electronic device 300, in particular between the proposing electronic computer and the first electronic device 300 operatively associated to the first patient subject 100.

In a preferred and non-limiting embodiment, said logic channel is kept open for a predetermined time determined by the retransmission of electronic data from the electronic device 300 towards the proposing electronic computer, and in particular at least for the time necessary to allow the patient to enter the electronic data required on the electronic device 300 and, optionally, it is an encrypted-type or otherwise protected logic channel, in order to preserve the privacy of the user's data. The proposition of the user interface from the proposing electronic computer towards the electronic device 300 is preferably carried out after a registration of the patient subject 100 and/or of the electronic device 300 operatively associated to the patient subject itself on the distributed computing environment, in particular on a database which can be operatively accessible by the first electronic computer 20 and/or by the second electronic computer 40. Thanks to this aspect, the user interface is selectively proposed towards a specific electronic device 300 operatively associated to the specific patient subject 100 with which the evaluating subject 200 wants to remotely interact.

The presence of a database and the possibility of interaction with the distributed computing environment after the introduction of access credentials on the electronic device 300 make the distributed computing environment described herein a closed-type computing environment, i.e. a computing environment defining a closed- and private-type logic network for data transceiving, not freely accessible to the public, wherein the interaction of one or more patient subjects 100 with one or more evaluating subjects 200 occurs only after a phase of introduction of access credentials and verification of the correctness thereof, this verification being performed automatically by the distributed computing environment itself after said phase of introduction.

The proposition of the user interface can preferably take place by means of a direct data transmission, and/or by means of a data transmission on a local network, and/or by means of a data transmission on a WAN network. Thanks to this aspect, the proposition of the user interface has no territorial limitation given by the distance between the electronic device 300 and the first electronic computer 20.

Trial and/or clinical electronic data can be managed through the user interface, and comprise at least one of the data comprised in the following list: a datum of motivation for consuming the drug, a datum of localization of the pain felt by the patient subject 100, a datum comprising the number of capsules or in any case the number of elements of drug consumed by the patient subject 100, a datum comprising the level of pain perceived by the patient subject before starting the treatment and with the aforementioned drug, a datum which comprises the level of pain perceived by the patient subject 100 following the beginning of the treatment with the aforementioned drug and in particular a pain perceived at predefined time instants starting from the beginning of the treatment with the drug, a datum of general satisfaction of the patient subject 100 at the end of the treatment with said drug, a datum of consumption of further drugs or in any case different drugs than the drug in question, in particular drugs with anti-inflammatory action, a datum comprising a level of pain perceived by the patient subject 100 following the end of treatment with the drug, a datum which comprises a number of elements of drug consumed by the patient subject before the start of the treatment with the drug, a datum comprising the potential presence of adverse events, and in particular serious adverse events, a datum which comprises the number of days and time in which the drug is consumed, and a datum which comprises the number of drug consumptions for off-label applications. The term "off-label" refers to any use of a pharmaceutical drug that is out of the conditions authorized by the designed authorities for pathology, patient population or dosage.

In particular, the level of perceived pain is expressed by the patient subject 100 through the introduction of an electronic datum on its electronic device 300. This electronic datum is, preferably but not-limiting thereto, a purely numerical electronic datum (for example expressed on a scale comprised between a first level 0 corresponding to the absence of pain and a second level 10 corresponding to the maximum imaginable and/or bearable pain) and is a datum measured on a predefined value VAS scale. Thanks to the use of a VAS scale, it is possible to provide a feedback on the level of perceived pain according to a standard uniformly accepted by the medical and pharmaceutical community.

In the previous portion of the description, it was observed that the data present in the user interface proposed to the patient subject 100 can comprise data relating to the occurrence of serious adverse events. For the purposes of this disclosure, "serious adverse events" (SAE) mean the occurrence, during the participation in a clinical trial, of any adverse effect resulting in one or more of the consequences of the present list: a death of the patient subject 100, an optionally actual and/or immediate risk to the life of the patient subject 100, a hospitalization of the patient subject 100, or in any case a prolongation of the existing hospitalization of the patient subject 100, a persistent and/or significant disability or in any case incapacity of the patient subject 100, a congenital anomaly and/or a birth defect, an operation to prevent a damage, in particular permanent, in any case an impairment of the patient subject 100. For the purposes of the present disclosure, it is not to be considered as "serious adverse event" an effect or event that hypothetically could have caused death if it had been more severe.

Having observed the effect that serious adverse events can have on the continuation of the life of the patient subject 100, as described more in detail in the following portion of the description, the distributed computing environment described herein is in particular configured to allow the sending of a specific alarm signal when the first patient subject 100 signals, through its first electronic device 300, the occurrence of a serious adverse event. The sending of an alarm signal is - in a preferred and non-limiting embodiment, a sending of an alarm signal automatically executed, preferably but non-limiting thereto, towards the evaluating subject 200 and/or to further subjects.

First of all, in order to establish the exact beginning of the treatment with a drug, the distributed computing environment described herein is configured to allow the definition and the storage in a memory operatively accessible by the first electronic computer 20 and/or by the second electronic computer 40, a first time instant t0 that corresponds to the time instant of the beginning of the treatment. By defining the first time instant t0, it is also possible to define the predefined time instants in correspondence of which the proposing electronic computer transmits towards the electronic device 300 operatively associated to the patient subject 100 a request for the transmission of an electronic datum relating to the level of pain currently perceived by the patient subject itself. Thanks to this aspect it is possible to know in a simple and effective way the levels of pain perceived with a temporal accuracy which is not achievable with the known art and it is, therefore, possible to obtain data on the efficacy of particular drugs whose activity and/or half-life are particularly short or long, not having to rely on an exclusive mnemonic recollection of the patient, which could be inaccurate.

Preferably but non-limiting thereto, the predefined time instants can be associated to at least a second time instant t1, or to one or more subsequent time instants t2, t3, t4, for example substantially equal to 30 min, 1 h, 2 h, 4h from the consumption of the drug (this consumption corresponding to the time instant t0).

Operatively, according to a predefined analysis protocol and/or according to a predefined data processing scheme, the pharmaceutical company 400 and/or the evaluating subject 200 define and/or store in the memory the second, third, and fourth instant of time t2, t3, t4, so that the distributed computing environment can perform an electronic calculation of a time interval between the first time instant t0 and the subsequent time instants as defined above, in order to allow the generation of a predefined electronic datum through which the electronic device 300 can exhibit, on its user interface, the possibility of introducing data relating to the pain perceived by the patient subject 100.

The phase of the reception of electronic data from the electronic device 300 operatively associated to the patient subject 100 comprises a phase of electronic filtering which can be performed, by way of example and non-limiting thereto, by the first electronic computer 20. This phase of electronic filtering has the purpose of filtering the electronic data received by the electronic device 300 in accordance with a predetermined risk criterion. It has already been previously written how in the phase of proposition and/or generation of the user interface, messages of identification of adverse events are proposed to the first patient subject 100. In particular, therefore, the phase of electronic filtering can comprise a distinction between electronic data relating to the occurrence of serious adverse events on the patient subject 100 with respect to the electronic data which do not comprise the aforementioned serious adverse events.

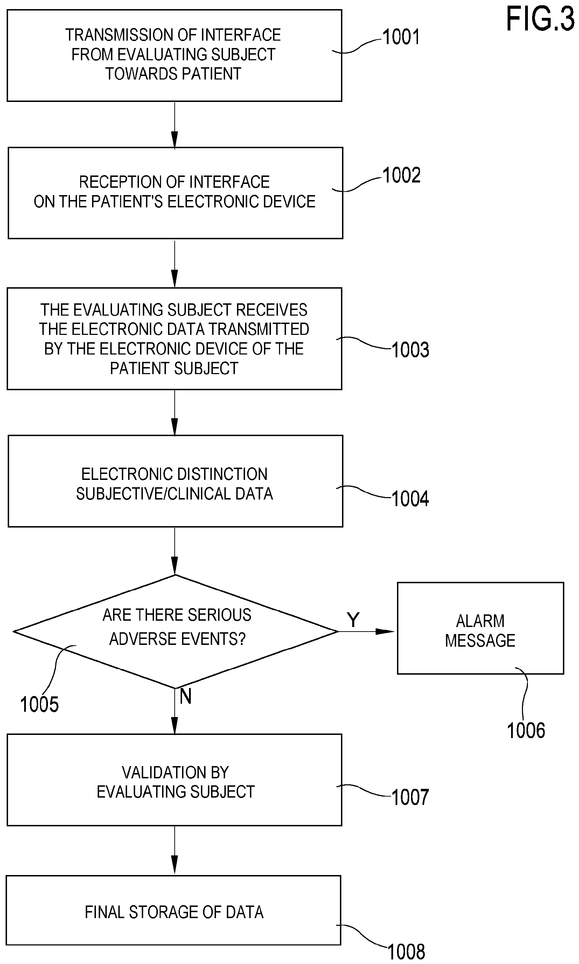

The first electronic computer 20 can therefore be configured to perform a phase of electronic filtering of the electronic data received by the electronic device 300, such that in case these received electronic data comprise data of occurred serious adverse events, it is generated an automatic, and in particular immediate transmission of:
- a priority alerting message (identified in the block diagram of figure 3 with the reference number 1006) towards the pharmaceutical company 400, in particular towards the second electronic computer operatively associated to the pharmaceutical company 400, and/or
- a priority alerting message towards the evaluating subject 200, in particular towards the first electronic computer operatively associated to said evaluating subject, and/or
- a priority alerting message towards a medical reviewer or a project manager 500, in particular towards a third electronic computer 50 operatively associated to the medical reviewer or project manager 500.

In addition or as an alternative to the above, an immediate electronic proposition of an emergency user interface towards the electronic device 300 can be provided, so that the user subject 100 can transmit further electronic detailed data relating to the serious adverse events towards the evaluating subject 200 and/or the pharmaceutical company 400 and/or the medical reviewer or project manager 500. This aspect is particularly useful as it allows healthcare professionals and/or the evaluating subject 200 to have a clearer picture of which actual side effects the drug may have had on the patient subject 100 and also allows, if it is the case, to immediately alert the emergency services for the treatment of the patient subject 100 in case of particularly serious side effects.

The distributed computing environment which is object of the present disclosure is also configured to transmit a particular user interface at the time of the conclusion of the treatment with said drug. In this case, the proposing electronic computer transmits to the electronic device 300 operatively associated to the patient subject 100 at least a datum requesting whether the patient subject 100 has assumed further drugs for the treatment of the pain, a datum requesting the degree of subjective satisfaction with the consumption of the drug, a datum requesting whether the patient subject 100 had to contact the doctor or pharmacist to solve one or more health problems concerning or connected to the pathology for which the patient subject 100 consumes a drug, or a datum confirming the consumption of the drug. The latter data is preferably presented on the user interface only when the patient subject 100 has never transmitted any electronic data relating to the consumption of the drug itself.

The phase of electronic validation carried out by the evaluating subject 200 through its own first electronic computer 20 on the electronic data transmitted by the patient subject 100 is characterized by the transmission of an electronic command confirming the correctness or applicability of at least part of the electronic data which are part of the first type of electronic data, i.e. the strictly clinical data required for the trial. By means of the electronic control, the evaluating subject 200 causes the final storage of a part of these data of the first type of electronic data which is considered correct or in any case validated. The electronic data transmitted by the patient subject 100 through its own electronic device 300 and being part of the second type of electronic data, subjective data, are not validated by the evaluating subject 200. These data, in a non-limiting embodiment, can be directly stored on the second electronic computer 40 associated to the pharmaceutical company 400, in particular not being transmitted by the first electronic computer 20. Alternatively, in a further non-limiting embodiment, the electronic data of the second type are simply displayed through a user interface on the first electronic computer 20 and are never transmitted to the second electronic computer 40.

The electronic confirmation command given by the evaluating subject 200 through their own first electronic computer 20, therefore, causes a final storage of this correct part of the electronic data in a memory 42 which is operatively associated or in any case operatively accessible by the second electronic computer 40.

In other terms, the data validated by the evaluating subject 200 are made available to the pharmaceutical/third party company 400 and in particular are stored on an electronic computer - namely, the second electronic computer 40 described herein - associated with it.

The memory 42 is therefore a memory containing a plurality of electronic data relating to a plurality of patient subjects 100, and is therefore a memory whose access in reading mode must be limited in order to guarantee the right level of privacy for each patient subject 100. For this reason, the distributed computing environment which is the object of the present disclosure defines an accessibility subnet to the memory 42 operatively accessible by and/or associated to the second electronic computer 40. In detail, the subnet S of accessibility is a logic-type subnet, and is characterized by a different extension depending on whether it is analyzed, or taken into consideration, the reading or, alternatively, the writing on the memory 42. In particular, in writing mode, the subnet S of accessibility to the memory 42 comprises the first electronic computer 20, the memory 42 itself, and the second electronic computer 40. This accessibility subnet explicitly excludes at least the first electronic device 300 associated to the first patient subject 100. In a preferred and non-limiting embodiment, the subnet S includes, in writing mode, exclusively only the first electronic computer 20, the memory 42 and the second electronic computer 40. It should be noted that figure 1 shows precisely with a discontinuous line the configuration in writing mode of the subnet S.

The subnet S of accessibility to the memory 42 operatively accessible by and/or associated to the second electronic computer 40, in reading mode, comprises the second electronic computer 40, the memory itself and excludes at least the first electronic device 300 associated to the first patient subject 100. In a preferred and non-limiting embodiment, this subnet S comprises exclusively the memory 42 and the second electronic computer 40. In other terms, while for the first electronic computer 20 operatively associated to the evaluating subject 200 it is possible to write electronic data on the memory 42, when written, they cannot any longer be read through the first electronic computer 20.

The block diagram in figure 3 schematically summarizes the operation of the method of collecting trial and/or clinical data which is also part of the present disclosure.

A first block, indicated with the reference number 1001, concerns a phase of transmission of the user interface from the electronic computer 20 of the evaluating subject 200 towards the electronic device 300 of the patient subject 100. The phase of transmission of the user interface 10 from the electronic computer 20 of the evaluating subject 200 towards the first electronic device 300 operatively associated to the first patient subject 100 is typical of a "web-based"-type configuration; in case the application is equipped with appropriate elements to provide by itself at least for the presentation of the user interface 10, the phase indicated with the reference number 1001 is actually a phase of generation of a user interface on the electronic device 300 and is preferably autonomously performed when the software application is executed.

Before the phase of transmission of the user interface, or of the generation of the user interface, identified by the block 1001, there are previous phases not represented in the figure, among which at least a phase of creation of a database operatively accessible by the first and/or by the second computer 20, 40, so that the database is configured to uniquely identify a specific patient subject 100, in particular the aforementioned first patient subject 100, for example through access credentials entered on the first electronic device 300. A phase of access to the distributed computing environment described herein is performed before the phase of transmission of the user interface, and following the phase of database creation. It should be noted that the access to the distributed computing environment described herein can be simultaneously performed by a plurality of patient subjects 100, each electronically identified by means of their own credentials, so that it is possible to establish a logical channel between the aforementioned proposing electronic computer and each of the electronic devices 300, each of which is operatively associated to a respective patient subject 100. Therefore, it will be possible to have also more logical channels operatively simultaneously present in an active way on the distributed computing environment described herein, but the transmission of the user interface described above will always be a selective-type transmission performed by the proposing electronic computer towards a specific electronic device 300 associated to a specific patient subject.

A subsequent phase, present especially when there is the aforementioned "web-based" application, is represented by the reception of the user interface transmitted by the first electronic computer 20 on the electronic device 300 of the patient subject 100. In figure 3 this phase is represented by the block identified by the reference number 1002.

This phase is followed by a phase of waiting, wherein the electronic computer 20 sets itself in a receiving configuration to allow the patient subject 100 to introduce the electronic data onto its own electronic device 100 and transmit them back to the electronic computer 20. This phase is represented in the block diagram of figure 3 with the reference number 1003.

When the electronic data reach the second electronic computer 20, a phase of electronic distinction (block 1004, figure 3) takes place between the first type of clinical electronic data and the second type of subjective data. The data of the second type, as described above, are not validated by the evaluating subject 200. The subsequent phases indicated in the remaining blocks of the diagram of figure 3 are carried out only for the first type of clinical electronic data.

If among the electronic data of the first type there are electronic data relating to serious adverse events (block 1005, output Y), an alarm message is transmitted, preferably by the first electronic computer 20. The transmission of the alarm message corresponds to the block identified by the reference number 1006. Otherwise, if among the data of the first type, there are no data relating to serious adverse events (block 1005, output N), these data are validated by the evaluating subject 200 without the need of transmitting alarm signals.

The phase of evaluation by the evaluating subject is schematically represented by the block 1007 in figure 3. The validation phase can be operatively represented by a click performed on a user interface operatively associated to the first electronic computer 20, and the part of the data that has been subjected to the aforesaid click can be transmitted to the memory 42. The part of the data that has not been subjected to the aforementioned click is therefore to be understood as a part of data not validated by the evaluating subject 200. In a preferred and non-limiting embodiment, this part of data is subsequently deleted. The electronic data provided by the patient subject 100 which reach the electronic computer 20 operatively associated to the evaluating subject 200 are temporarily stored in a memory 12 operatively accessible by the electronic computer 20 itself. The abovementioned phase of identification for the non-validated data comprises therefore a separate storage in the memory 12.

The portion of the first type of clinical electronic data that has been validated by the evaluating subject 200 and finally definitively memorized on a memory 42 operatively accessible by the second electronic computer 40, in the diagram of figure 3, is schematically represented by block 1008.

The Applicant points out that through the method described herein it is possible to displace the various elements that are part of the distributed computing environment also in geographically distinct areas and it is therefore easily possible to perform a collection of trial and/or clinical data without the pharmaceutical company 400, the evaluating subject 200 and the patient subject 100 being all in close proximity to each other. For example, through the method and the distributed computing environment described herein, it is possible to avoid onerous appointment commitments between the patient subject 100 and the evaluating subject 200.

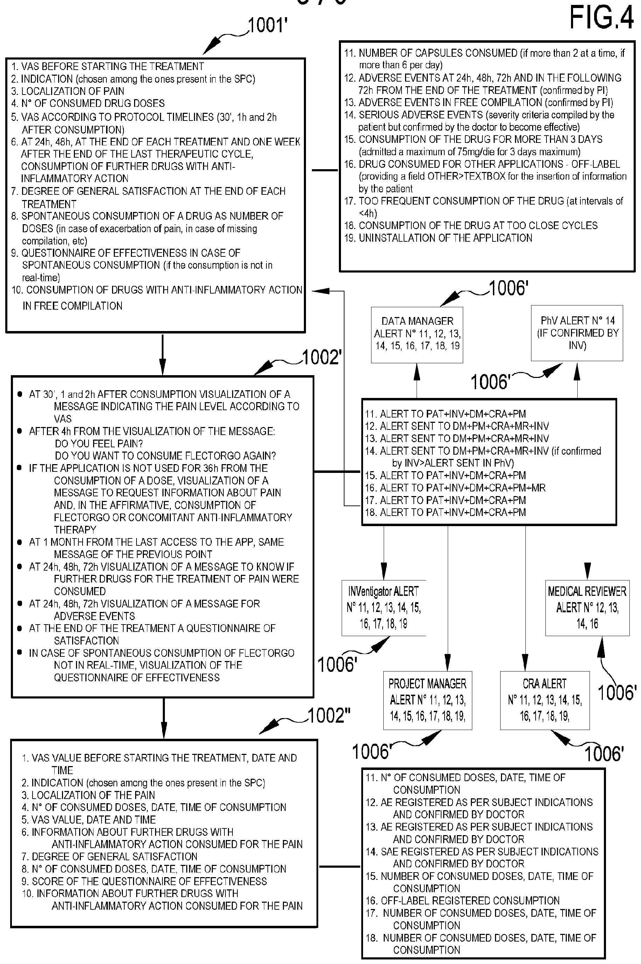

Figure 4 shows a data flow diagram between the patient subject 100 and the software application installed on its own electronic device 300 and/or the first electronic computer 20. In figure 4, in order to better identify the data flows directed towards one or more users, arrows with different lines have been used; in particular, the data flows directed from the users (first patient subject 100, in particular, through its first electronic device 300) towards the distributed computing environment are identified by a thick arrow, while the data flows directed from the distributed computing environment towards the users themselves are identified by a thin arrow.

The block 1001' comprises a plurality of data which are transmitted by the patient subject 100 towards the software application installed on board of the electronic device 300:
- (reference 1) level of pain according to the VAS scale at the beginning of the treatment (time instant t0);
- (reference 2) indication according to which it was decided to consume the drug;
- (reference 3) localization of pain;
- (reference 4) number of capsules consumed by the patient subject 100;
- (reference 5) level of pain, in particular according to the VAS scale, at pre-established time intervals (30 min, 1 h and 2 h starting from the time instant t0);
- (reference 6) possible consumption of further drugs with anti-inflammatory action, in particular at 24h, 48h, and at the end of each treatment and one week after the end of the last therapeutic cycle;
- (reference 7) degree of general satisfaction of the patient subject 100 at the end of the treatment with the drug;
- (reference 8) any spontaneous consumption of the drug (for example in case of exacerbation of pain);
- (reference 9) evaluation of the efficacy of the treatment in case of spontaneous consumption;
- (reference 10) possible consumption of anti-inflammatory drugs, through free filling in the data field;
- (reference 11) number of capsules consumed as a total during the treatment, in particular if more than two at a time and/or if more than 6 per day;
- (reference 12) potential presence of adverse events at predefined time intervals (for example 24, 48 and 72h) after the beginning of the treatment;
- (reference 13) potential presence of adverse events in free compilation;
- (reference 14) potential presence of serious adverse events;
- (reference 15) possible consumption of a drug over a temporal duration greater than or equal to three days;
- (reference 16) possible consumption of the drug for off-label applications;
- (reference 17) possible consumption of the drug at intervals lower than 4 hours;
- (reference 18) possible consumption of the drug at too close cycles;
- (reference 19) data regarding the possibility of uninstallation of the application from the electronic device 300.

The Applicant points out that particularly with respect to the indication of the potential presence of adverse events (reference 12 and 13), there is the possibility for the patient subject 100 to have a free text filling in a text data field provided through the user interface proposed on the electronic device 300.

The reference block 1002' indicates the operations performed by the software application and/or by the first electronic computer 20 with reference to the data expressed in the block 1001'. An operation can consist, at predetermined time instants (30 min, 1, and 2 h starting from the time instant t0), in displaying a message on the electronic device 300 of the patient subject 100 so that the latter transmits an electronic data relating to the level of pain (preferably on a VAS scale) after consumption. A further operation can consist in the transmission, preferably after 4 hours after consuming the drug, of a message transmitted towards the first electronic device 300 so that the latter sends back, by means of the action of the first patient subject 100, an electronic datum relating to the possible consumption of an additional element of drug, for example an additional tablet.

If a dosage ratio is defined between the element of drug and the patient subject 100, upon reaching a maximum dosage limit, the first electronic computer 20 and/or the application installed on the electronic device 300 can cause the display of an alerting message to signal the reaching of a maximum drug consumption limit, by way of example and non-limiting thereto, a maximum daily dose limit of the drug.

A further operation can be the transmission of a reminder message towards the electronic device 300, if the patient subject 100 has not transmitted, through its own electronic device 300, any electronic data, in particular neither of the first type nor of the second type.

At the end of the treatment, further operations can comprise the proposition of a user interface that causes a request for data transmission towards the first computer 20 in order to identify whether the patient subject 100 has consumed other drugs for the treatment of pain and/or the degree of satisfaction with the consumption of the drug and/or whether it considers the drug consumed more or less effective than the drugs consumed for the treatment of the same disease and/or the same symptom, and/or whether it was necessary to consult a doctor and/or pharmacist during the treatment with the drug.

It should be noted how block 1002' also identifies a plurality of actions to be taken which cause a data flow from the first electronic computer 20 and/or from the application installed on the electronic device 300 towards external devices. In particular, what can cause such data flow are the following events:
- (reference 11) number of capsules consumed as a total during the treatment;
- (reference 12 and 13) potential presence of adverse events;
- (reference 14) potential presence of serious adverse events;
- (reference 15) potential consumption of a drug over a temporal duration greater than or equal to three days;
- (reference 16) potential consumption of the drug for off-label applications;
- (reference 17) potential consumption of the drug at intervals lower than 4 hours;
- (reference 18) potential consumption of the drug at too close cycles;
- (reference 19) potential uninstallation of the application from the electronic device 300.

In particular, the data referred to in references 11, 12, 13, 14, 15, 16, 17, 18, 19 are transmitted towards a "data manager" subject and/or towards a clinical controller subject (CRA, "Clinical Research Associate"), and/or towards a "project manager" subject.

The "data manager" subject is a subject whose role belongs to a third-party company with respect to the pharmaceutical company, and in the case of the schematic representation of figure 1 can be made to correspond to the subject indicated by reference 500. The company to which the "data manager" subject refers is a CRO (Contract Research Organization) and is responsible, on behalf of the sponsor and/or of the client, for the collection and/or management and/or storage of data relating to a clinical trial.

The "project manager" subject is a subject associated to a third-party company with respect to the pharmaceutical company, and is responsible, on behalf of the sponsor and/or of the client, for supervising all the operational aspects relating to a given clinical trial (typically involving various clinical trial centers) in order to ensure that the trial is overall conducted in compliance with the specifications of the clinical study protocol, quality international standards and applicable regulatory requirements.

The clinical controller is a figure associated to a third-company with respect to the pharmaceutical company 400, and is typically associated with a CRO. Its role, on behalf of the sponsor and/or of the customer, is to keep the contact with the investigators (both remotely, and in person, for example by going to clinical trial centers) to ensure that the testing at the clinical trial center is conducted in compliance with the specifications of the clinical study protocol, quality international standards and applicable regulatory requirements. In summary, therefore, the clinical controller ensures the supervision and a support with an operational profile towards investigators, for the conduct of a determined clinical trial. The clinical controller can be responsible for ensuring the veracity of the data collected by the investigators.

In particular, electronic data are sent to a medical reviewer if one or more of the cases referred to in references 12, 13, 14 or 16 occur, as the potential presence of serious adverse events must be notified to an approved physician. The "medical reviewer" subject is a subject associated with a CRO (Contract Research Organization) who is responsible, on behalf of the sponsor and/or of the client, for a strictly medical review of the clinical data collected by the investigator; in particular, the "medical reviewer" subject evaluates the completeness and/or adequacy and/or consistency (always from a strictly medical/clinical point of view). Electronic data are sent to the pharmaceutical company 400 (or alternatively to a pharmacovigilance company, which as in the case of the attached figures can be indicated in short with the acronym PhV) if entries of electronic data referred to in reference 12 and 14 or 16 occur. In detail, the pharmacovigilance function indicated with the reference PhV, besides the pharmaceutical company 400, can correspond to a specialized company, in charge of drawing up any necessary notifications to the competent authorities responsible for the management of serious adverse events (SAE) recorded during a clinical trial and adverse events possibly related to the use of a determined drug reported by users (doctors, pharmacists, patients). Electronic data, for example at least part of the first type of clinical trial electronic data, are sent to a data manager and/or to a project manager if data entries occur in accordance with the above mentioned reference 11 and/or 12 and/or 13 and/or 14 and/or 15, and/or 16, and/or 17, and/or 18, and/or 19. It should be noted that in the figure, the subject indicated as "investigator" corresponds to the evaluating subject 200 indicated in this description, therefore a doctor and/or a dentist identified by the pharmaceutical company for the identification, the recruiting, the follow-up and the collection of clinical data according to the requirements of the clinical research protocol. The transmission of electronic data towards said third parties comprises the transmission of an alarm signal and is identified in figure 4 by reference number 1006'.

In a particular embodiment, the computing environment described herein can be configured to cause the sending of an electronic notification or proposition of a check-up visit at a doctor's office to the electronic device 300. Such notification or proposition can be sent at predefined time intervals, for example and preferably every 3 months, or alternatively every 4 months or every 2 months. The sending of the electronic notification or proposition is carried out by the electronic computer identified herein as the proposing electronic computer. On the software application, this electronic notification or proposition causes the generation of an alerting message which can conveniently be of an audible or visual type (by way of example and non-limiting thereto, a pop-up).

In other terms, the distributed computing environment described herein can be configured to receive electronic data (phase identified in figure 3 by the reference number 1003) from the first electronic device 300 operatively associated to the first patient subject 100, and in accordance with a predetermined distribution scheme, cause a selective, optionally automatic and/or immediate retransmission of at least part of the electronic data received, to a plurality of distinct recipients; in particular, the distributed computing environment described herein can be configured to cause the retransmission of a same part of the received electronic data towards at least a first and a second recipient.

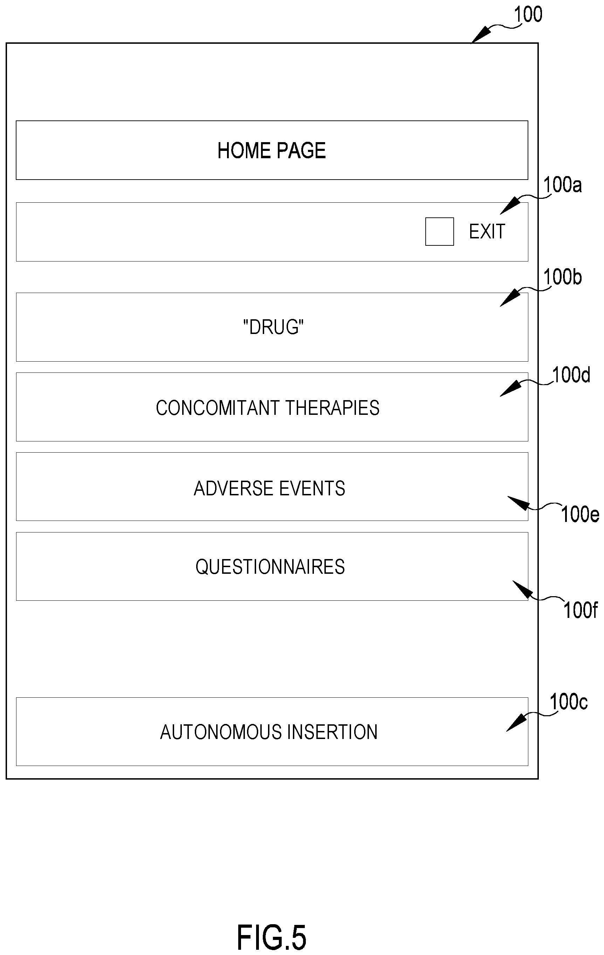

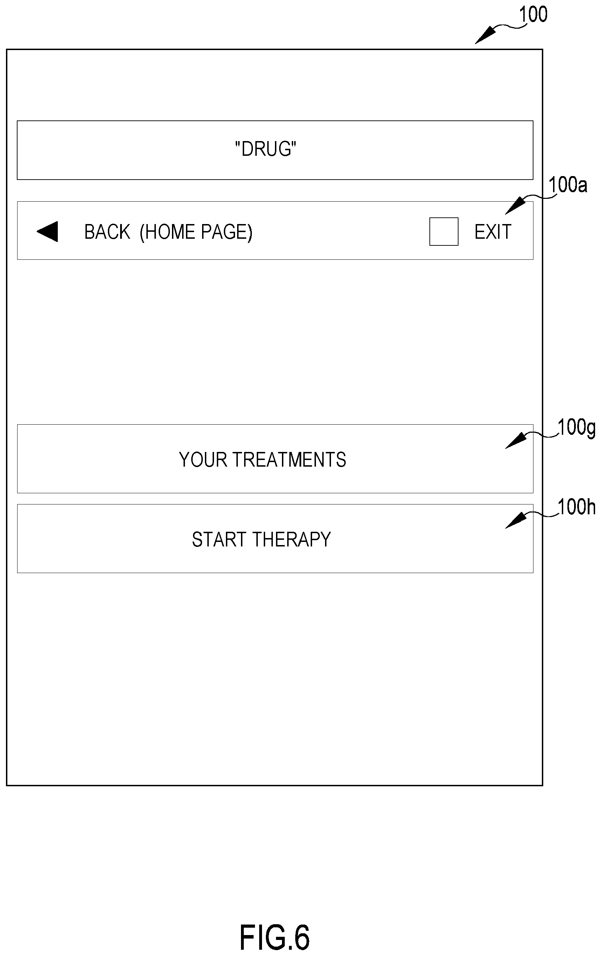

Figure 5 and figure 6 show two non-limiting screens of the user interface proposed to the electronic device 300 associated to the patient subject 100. In particular, figures 5 and 6 represent two screens represented alternatively on a screen of the electronic device 300.

In a non-limiting embodiment, the user interface proposed by the proposing electronic computer is of hierarchical type, i.e. such that the patient subject 100 is allowed to work on selection structures nested among themselves and alternatively and/or selectively selectable by the action on buttons, therefore with an at least partially guided, not free, alternative of selection and/or data entry. Thanks to this aspect, the navigation of the user interface is simplified, and the risk of data entry errors is reduced with respect to what would happen in case the user interface was fully in free text data field.

In particular, figure 5 shows a main screen where there is, first of all, a data field for selecting a specific type of drug, by way of example but non-limiting thereto, nimesulide or acetylsalicylic acid. This data field is identified by the numeric reference 100b. Further fields that can be present in the aforementioned main screen are, in a non-limiting embodiment, a data field 100d indicating the presence of concomitant therapies, a data field 100e indicating the presence of adverse events and a data field 100f indicating a possible questionnaire. Each of the data fields 100b - 100f represents a virtual button clickable by the patient subject 100 through the user interface proposed on its electronic device 300. On the main screen there is also an exit button 100a, which, for example, allows to exit the application and close and/or stop the execution of the application itself and a button for autonomous insertion (indicated in figure 5 with the reference number 100c) for retrospective rather than real-time data recording.

Figure 6 shows a screen alternatively represented on the monitor of the electronic device 300: in particular, this screen appears when the user clicks on the data field 100b relating to the selection of the specific type of drug. This screen shows, in a non-limiting way, the presence of a first button relating to the history of what was recorded up to that moment by the patient subject in relation to the drug consumption (button identified by the reference 100g, "your treatments") and the presence of a second button (identified by the reference 100h, "start therapy"), alternatively selectable instead of the first button, from which the patient subject 100 can proceed with the insertion of data relating to the beginning of a therapy cycle with the drug. In a non-limiting embodiment, the click performed on the button 100h determines the transmission of an electronic datum of the beginning of the drug consumption from the electronic device 300 towards the first electronic computer 20. In a non-limiting embodiment, such transmission occurs immediately at the clicking, so that the evaluating subject 200 is promptly informed of the drug consumption by the patient subject 100.

In summary, therefore, a particular software application can be installed on the electronic device 300 which, preferably but non-limiting thereto, can be operatively associated to a private profile of the patient subject 100, in order to guarantee privacy. The patient subject can access the software application by entering its access credentials or, alternatively, by using biometric data, such as, by way of example but non-limiting thereto, the acquisition of a digital fingerprint through the electronic device 300 itself. The software application is specifically configured to cooperate with the other elements of the distributed computing environment described herein in order to allow the evaluating subject 200 to properly carry out the task of collecting the first type of trial and/or clinical data from the patient subject 100.

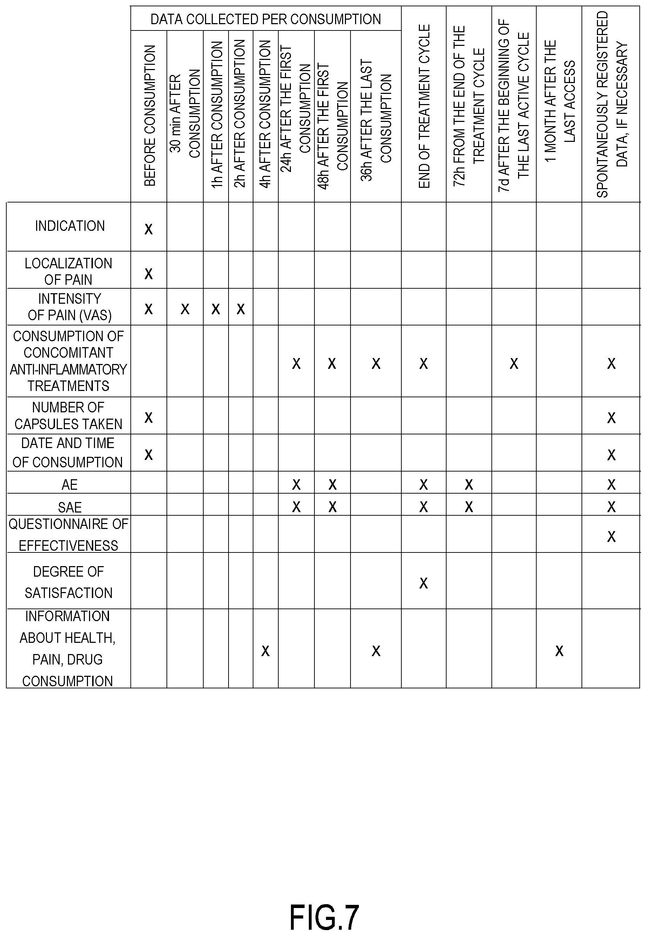

The table of figure 7 shows an association between particular indications provided by a user subject on moments associated with the consumption of a drug. This table is provided for summary purposes. Among the collected data there are:
- before the consumption of the drug, an indication, a localization of the pain, a pain intensity, particularly but non-limiting thereto, according to a VAS scale, a number of capsules or in any case of elements of drug consumed, the date and time of the consumption;
- at a time t1, preferably substantially corresponding to 30 minutes following the first consumption, an indication of pain intensity, particularly but non-limiting thereto, according to a VAS scale;
- at a time t2, subsequent to t1 and preferably substantially corresponding to 1h following the first consumption, a further indication of pain intensity, particularly but non-limiting thereto, according to a VAS scale,
- at a time t3, subsequent to t1 and t2 and preferably substantially corresponding to 2h following the first consumption, a further indication of pain intensity, particularly but non-limiting thereto, according to a VAS scale;
- at a time t4, subsequent to the previous t1, t2, t3 and preferably substantially corresponding to 4h following the first consumption, information on the state of health, pain (intensity, particularly but non-limiting thereto, according to a VAS scale), and on the consumption of the drug;
- at a time t5, subsequent to the previous t1, t2, t3, t4 and preferably substantially corresponding to 24h following the first consumption, a first set of information relating to the concomitant consumption of painkillers with the drug, the possible presence of adverse events (AE) and the possible presence of serious adverse events (SAE);
- at a time t6, subsequent to the previous t1, t2, t3, t4 and t5, and preferably substantially corresponding to 48h following the first consumption, a second set of information relating to the concomitant consumption of painkillers with the drug consumed, the possible presence of adverse events (AE) and the possible presence of serious adverse events (SAE);
- at a time t7, subsequent to the previous t1 - t6, and preferably substantially corresponding to 36h following the last consumption, information relating to the concomitant consumption of painkillers with the drug consumed, and information about health condition, pain (intensity, particularly but non-limiting thereto, according to a VAS scale), and the consumption of the drug;
- at a time t8, subsequent to the previous t1 - t7, and preferably substantially corresponding with the end of the treatment with the drug, information relating to the concomitant consumption of painkillers with the drug consumed, information relating to the occurrence of adverse events (AE) and of serious adverse events (SAE), as well as the level of general satisfaction of the patient subject 100;
- at a time t9, subsequent to the previous t1 - t8, preferably substantially corresponding to 72h following the end of the treatment with the drug, information relating to the occurrence of adverse events (AE) and serious adverse events (SAE);
- at a time t10, subsequent to the previous t1 - t9, preferably substantially corresponding to 7 days following the end of the last active cycle with the drug, information relating to the concomitant consumption of painkillers with the drug,
- at a time t11, subsequent to the previous t1 - t10, preferably substantially corresponding to 1 month after the last access, information about health condition, pain (intensity, particularly but non-limiting thereto, according to a VAS scale), and on the consumption of the drug.

With regard to the data regarding pain intensity according to the VAS scale before the consumption of the drug, preferably these data are recorded only if acquired in real-time.

With regard to the data regarding the concomitant consumption of painkillers at time t7, this information shall be collected only if the patient subject 100 expresses, through its user interface, an actual and/or residual presence of pain.

Further data can be recorded by the patient subject 100 even outside the previously mentioned time instants t1 - t11: in a preferred but non-limiting embodiment, these data comprise one or more pieces of information contained in the following list:
- information about the concomitant consumption of painkillers;
- a number of capsules or anyway other elements of drug consumed, and optionally the date and time of consumption;
- the occurrence of adverse events (AE) and/or serious adverse events (SAE);
- a questionnaire about the effectiveness of the treatment with the drug.

The software application comprises portions of code which, when executed by a data processing unit of the electronic device 300, first of all cause the appearance on a monitor of the electronic device 300 of at least a user interface, in particular an electronic user interface for entering clinical trial data, through which the patient subject 100 can enter electronic data by means of the electronic device 300 and/or select electronic data submitted through the user interface itself. The software application also comprises portions of software code which, when executed, cause the appearance of one or more data fields 11 into which the patient subject 100 can enter electronic data.

The software application described herein is also configured to cause a transmission of at least part of the electronic data entered and/or selected by the patient subject 100 by means of its electronic device 300 towards an electronic computer remotely operatively connected to the electronic device 300 by means of a logical channel, and is configured to cause a generation of at least one alerting or alarm signal, emitted by the electronic device 300, as a consequence of a reception of an alarm notification emitted by the electronic computer itself.

Preferably, although non-limiting thereto, the electronic computer remotely operatively connected to the electronic device 300 is precisely the first electronic computer 20 operatively associated to the evaluating subject 200, and even more preferably this first electronic computer 20 is the same first electronic computer 20 which caused the transmission of data towards the electronic device 300 itself.

The software application installed on the electronic device 300 is also configured to cause the transmission of a storage request signal of a first time instant t0 in a memory (in particular the memory 12 operatively associated to the first electronic computer 200 or the memory 42 operatively associated to the second electronic computer 400) and, as previously mentioned, this first time instant corresponds to the time instant in which the patient subject 100 selects by means of the electronic device 300 a control or button 100b of confirmation of the first consumption of a drug.

Through the software application, the generation of the alerting or alarm signal can be caused in correspondence with at least a subsequent predetermined time instant t1 elapsing from the first time instant t0; this at least a predetermined time instant t1 corresponds to a predetermined number of hours following the first time instant t0 in which the drug was consumed, and allows the evaluating subject 200 to determine at least part of the data useful for determining the drug efficiency on the patient subject 100. Following the transmission of the alerting or alarm signal, the electronic device 300, always by means of the operation of the software application, requests through the emission of a predetermined notification signal (visual and/or audible) an action by the patient subject 100, in particular the confirmation of a value datum of a datum of the level of perceived pain; in particular, this datum of the level of perceived pain is a purely numeric electronic datum, measured on a predefined value VAS scale.

In a non-limiting embodiment, the software application causes the showing of a user interface, such that to the patient subject 100 is presented a visual diagram with a linear scale comprised between a minimum value, corresponding to a substantial absence of pain, and a maximum value corresponding to a maximum value of imaginable pain, . On this scale there is a cursor, which can be moved by the patient subject (clearly through the interaction with the electronic device 300) between the aforementioned minimum value and the maximum value, for example by dragging the finger on the screen of the electronic device 300, if the screen is touch-sensitive. The software application can present, in conjunction with the visual diagram, a confirmation button, through which the user can electronically confirm the level of pain selected by using the virtual cursor, so that in the end it is possible to transform the level of pain selected with the cursor into a pure numeric value, to be transmitted, for example, automatically and immediately after pressing the confirmation button.

The software application can also be configured to allow the patient subject 100 to select the number of drug elements consumed by showing on the screen of the electronic device 300 at least a first addition button, by way of example and non-limiting thereto marked by an icon "+", which allows adding the number of drug elements consumed by the patient subject 100, and a second subtraction button, by way of example and non-limiting thereto marked by an icon "-", which allows subtracting the number of drug elements consumed by the patient subject 100. These buttons are therefore virtual buttons. In substantial correspondence with the first and second button, the software application advantageously allows viewing the current number of elements of drug consumed. The software application can present, in conjunction with the visual diagram, a confirmation button through which the user can electronically confirm the number of elements of drug consumed. In a non-limiting embodiment, the software application can be configured to cause a transmission of the electronic data relating to the number of elements of drug acquired directly towards the evaluating subject 200.

The user interface, of the graphic type, which allows the user to select the occurrence of serious adverse events deriving from the consumption of the drug, can comprise a first virtual button, which the patient subject 100 can press and/or select in case adverse events requiring medical care and/or a trip to an emergency room have occurred, and a second virtual button, which the patient subject 100 can press and/or select in case adverse events not requiring medical care and/or a trip to an emergency room have occurred. In a particular and non-limiting embodiment, the software application can be configured to display on the screen of the electronic device 300:
- a first virtual button, which the patient subject 100 can press and/or select if adverse events requiring medical care have occurred,
- a second virtual button, which the patient subject 100 can press and/or select if adverse events not requiring medical attention have occurred,
- a third virtual button, which the patient subject 100 can press and/or select if adverse events requiring a trip to an emergency room have occurred,
- a fourth virtual button, which the patient subject 100 can press and/or select if adverse events not requiring a trip to an emergency room have occurred.

If the patient subject 100 selects a button corresponding to the occurrence of an adverse event such that it has required a trip to the emergency room and/or medical care, the request for selecting the confirmation button may not be provided; thanks to this aspect, in case the serious adverse event has recently occurred and leads to a temporary disability or worse condition of the patient subject 100, the risk that the electronic data will not be transmitted from the electronic device 300 to the due recipient because of a sudden impossibility of the patient subject 100 is reduced.

As it can be seen from the previous portion of the description, the software application described herein allows providing a particularly simple graphic interface. Thanks to this aspect, the selection of the number of drug elements consumed is particularly easy and intuitive, and consequently the risk of error in data entering, even by inexperienced or elderly users, is reduced.

Parts of the process described herein can be obtained by means of a data processing unit, or control unit, technically replaceable with one or more electronic computers designed to execute a portion of a software or firmware program loaded on a memory medium. This software program can be written in any known type of programming language. The electronic computers, if equal in number to two or more, can be connected to each other through a data connection such that their computing powers are shared in any way; the same electronic computers can therefore be installed also in geographically different positions, realizing a distributed computing environment through the aforementioned data connection.

The data processing unit, or control unit, can be a general-purpose type processor specifically configured to execute one or more parts of the process identified in the present disclosure through the software or firmware program, or be a dedicated ASIC or dedicated processor or a FPGA, specifically programmed to execute at least part of the operations of the process described herein.

The memory medium can be non-transitory and can be internal or external to the processor, or control unit, or data processing unit, and can - in particular - be a memory geographically located remotely with respect to the electronic computer. The memory medium can also be physically divided into several portions, or be in the form of a "cloud", and the software or firmware program can physically be on, or provide, portions stored on portions of memory geographically divided among them.

The invention is not limited to the embodiments illustrated in the drawings. For this reason, it is understood that wherein features mentioned in the claims are followed by reference signs, such reference signs are included for the sole purpose of increasing the intelligibility of the claims, not constituting in any way a limitation of the claims themselves.

It is finally clear that additions, modifications or variants which are obvious for a person skilled in the art can be applied to the object of the present disclosure, without departing from the scope provided by the attached claims.

## Claims

1. A computer-implemented method for collecting trial and/or clinical data from at least a patient subject (100), the method comprising:
- a phase of proposing and/or generating a user interface (10) on a first electronic device (300) operatively associated to a first patient subject (100), wherein the user interface (10) comprises at least a data field (11) configured and destined to the introduction, by the first patient subject (100), of at least a first type of trial and/or clinical electronic data relating to the first patient subject (100);
- a phase of reception (1003) on a proposing electronic computer (20; 40) of at least said first type of electronic data relating to the first patient subject (100) from the first electronic device (300), and a subsequent phase of intermediate and/or temporary storage of at least part of said first type of electronic data of the first patient subject (100), so received, on an electronic memory (12, 42),
- an electronic validation phase (1007), performed by an evaluating subject (200) distinct from the patient subject (100) and subsequent in time to said phase of reception (1003), wherein the evaluating subject (200) performs a verification phase on at least part of said first type of electronic data of the first patient subject (100) and a phase of generation and/or transmission, through a first electronic computer (20) operatively associated and destined to the evaluating subject (200), of an electronic command of confirmation of correctness and/or applicability of at least a part of said first type of electronic data of the first patient subject (100),
said electronic command of confirmation causing a final storage (1008) of a correct part of said first type of electronic data of the first patient subject (100) for which the correctness and/or applicability has been electronically confirmed through the electronic command of confirmation.

2. Method according to claim 1, wherein the electronic command of confirmation causes:
- an electronic identification of a correct part of said first type of electronic data of the first patient subject (100);
- a final storage (1008) of the correct part of said first type of electronic data of the first patient subject (100) on the first electronic computer (20); or
- a final storage (1008) of the correct part of said first type of electronic data of the first patient subject (100) on a second electronic computer (40), different than the first electronic computer (20), optionally operatively associated to a pharmaceutical company (400).

3. Method according to claim 2, wherein:
- the electronic validation phase (1007) comprises, optionally ends with, an electronic transmission of the correct part of said first type of trial and/or clinical electronic data relating to the first patient subject (100) on the first electronic computer (20) and/or on the second electronic computer (40); the automatic transmission taking place automatically following the electronic command of confirmation, and wherein,
- following the electronic validation phase (1007), a non-correct part of said first type of electronic data of the first patient subject (100) is defined, wherein said non-correct part is a part of the first type of electronic data of the first patient subject (100) for which the electronic command of confirmation of correctness and/or applicability has not been transmitted, and said non-correct part is electronically identified and/or discarded and, optionally, is electronically identified and/or discarded without electronic erasing and/or is ended with a separate electronic archiving of the non-correct part of the first type of electronic data of the first patient subject (100).

4. Method according to one or more of the preceding claims, comprising a phase of electronic selection of at least the first electronic device (300), among a plurality of electronic devices (300) each one operatively associated to a respective patient subject (100), optionally through the first electronic computer (20), and the proposition and/or generation of a user interface (10) on the first electronic device (300) comprises a selective transmission of electronic data towards the first electronic device (300);
the selective transmission of electronic data towards the first electronic device (300) taking place between the proposing electronic computer (20; 40) and the at least a specific electronic device (300), and/or the electronic proposition of the electronic interface towards the specific electronic device (300) taking place between the proposing electronic computer (20; 40) and said first electronic device (300).

5. Method according to claim 4, wherein the selective transmission of electronic data towards the first electronic device (300) is preceded by a phase of setting-up a logical channel between the proposing electronic computer (20; 40) and the first electronic device (300); optionally the method comprising the keeping-up of the logical channel for a predetermined period of time of which the end of a retransmission of data from the electronic device (300) towards the proposing electronic computer (20; 40) determines a limit.

6. Method according to one or more of the preceding claims, comprising the definition of a distributed computing environment and/or a closed-type logic network of data transceiving, wherein the interaction of one or more patient subjects (100) with one or more evaluating subjects (200) takes place only following a phase of introduction of credentials of access and verification of the correctness thereof, at the moment of the introduction by the patient subject (100) and/or of the evaluating subject (200).

7. Method according to one or more of the preceding claims, wherein the first type of trial and/or clinical electronic data comprises at least one of the data of the following list:
- a datum of motivation for assuming a drug;
- a datum comprising the indication for which the patient subject (100) decides to start a treatment cycle;
- a datum of localization of pain felt by the patient subject (100);
- a datum comprising the number of capsules assumed by the patient subject (100);
- a datum comprising a level of pain perceived by the patient subject (100) before starting the treatment with a drug,
- a datum comprising a level of pain perceived by the patient subject (100) following the beginning of the treatment with a drug, optionally a pain perceived at substantially predefined time instants starting from the beginning of the treatment;
- a datum of general satisfaction of the patient subject (100) at the end of a treatment with a drug;
- a datum of consumption of further and/or other drugs with respect to said drug, optionally drugs with anti-inflammatory action;
- a datum comprising a level of pain perceived by the patient subject (100) following the end of a treatment by means of a drug;
- a datum comprising a number of elements of drug consumed by the patient subject (100) and/or an amount of drug consumed by the patient;
- a datum comprising the potential presence of adverse events;
- a datum comprising the potential presence of serious adverse events;
- a datum comprising the number of days in which the drug is consumed;
- a datum comprising consumptions of a drug for off-label applications;
- a datum comprising the potential consumption of the drug at intervals lower than 4 hours;
- a datum comprising the potential assumption of the drug at too close cycles;
and wherein the level of perceived pain is expressed and/or transmitted and/or introduced by the patient subject (100) as a numeric electronic datum, measured on a predefined value VAS scale.

8. Method according to one or more of the preceding claims, comprising a phase of electronic filtering (1005) of said first type of trial and/or clinical electronic data relating to the first patient subject (100) and received with said phase of reception (1003), wherein the electronic filtering (1005) takes place in accordance to a predetermined risk criterion, and wherein, in the phase of electronic filtering (1005), in case in the phase of reception that follows the proposition and/or generation of the user interface (10) towards the at least a patient subject (100), the patient subject (100) through the first electronic device (300) transmits an electronic datum relating to serious adverse events, the method comprises a phase of automatically transmission of:
- a priority alerting message (1006) towards a pharmaceutical company (400) and/or towards a third company, differing from said pharmaceutical company (400), in particular towards the second electronic computer (40) operatively associated to the pharmaceutical company (400), and/or of
- a priority alerting message towards the evaluating subject (200), in particular towards the first electronic computer (20) operatively associated to said evaluating subject (200), and/or of
- a priority alerting message towards a medical reviewer or a project manager (500), in particular towards a third electronic computer operatively associated to the medical reviewer or project manager (500);
and/or wherein the method comprises an immediate electronic proposition of an emergency user interface towards the first electronic device (300), so that the user subject (100) can transmit further electronic data of detail relating to serious adverse events towards the evaluating subject (200) and/or the pharmaceutical company (400) and/or the medical reviewer or project manager (500).

9. Method according to one or more of the preceding claims when depending on claim 4, wherein the electronic proposition (1001) of the user interface (10) takes place through a transmission of electronic data of a client-server type, wherein the proposing electronic computer (20; 40), in particular the first electronic computer (20) and/or the second electronic computer (40), acts like server and/or wherein the first electronic device (300) acts like a client, the first electronic device (300) being provided with a monitor, said electronic data being configured to cause the appearance on the monitor of at least a screen from which the patient subject (100) detects questions to answer through the introduction of electronic data inside at least a data field (11).

10. A distributed computing environment, comprising:
- at least a proposing electronic computer (20; 40), configured for causing a proposition and/or generation of, and/or an operative association with, a user interface (10) towards at least a first electronic device (300) operatively associated to a first patient subject (100) and remotely positioned with respect to the proposing electronic computer (20; 40), wherein the user interface (10) comprises at least a data field (11) configured and destined to the introduction, by the first patient subject (100), of at least a first type of trial and/or clinical electronic data;
- at least a first electronic computer (20) operatively associated to an evaluating subject (200) differing from the patient subject (100), and an electronic memory (12, 42) operatively accessible by the first electronic computer (20), wherein the first electronic computer (20) is configured for receiving (1002) at least said first type of trial and/or clinical electronic data transmitted by the patient subject (100) through the electronic device (300), and is further configured for causing an intermediate and/or temporary storage of the received electronic data on the electronic memory (12, 42),
wherein:
- the first electronic computer (20) is configured for causing an electronic validation (1007), operatively performed by the evaluating subject (200), the electronic validation (1007) being subsequent in time to the reception (1003) of the electronic data, wherein the evaluating subject (200) performs an action of verification on at least part of the first type of trial and/or clinical electronic data of the first patient subject (100) and wherein the first electronic computer (20) is configured for transmitting an electronic command of confirmation of correctness and/or applicability of at least a part of said first type of electronic data of the first patient subject (100), said electronic command of confirmation causing a final storage (1008) of a correct part of said first type of electronic data of the first patient subject (100) for which the correctness and/or applicability has been electronically confirmed through the electronic command of confirmation.
